# EUROPEAN PATENT SPECIFICATION

(11) **EP 2 429 627 B1**
(45) Date of publication and mention of the grant of the patent: **14.06.2017**
(21) Application number: 10767862.5
(22) Date of filing: 23.04.2010
(51) Int. Cl.: A61M 37/00

(54) **METHODS FOR MANUFACTURING MICROPROJECTION ARRAYS**
VERFAHREN ZUR HERSTELLUNG VON MIKROPROJEKTIONSARRAYS
PROCÉDÉS DESTINÉS À LA FABRICATION DE RÉSEAUX DE MICROSAILLIE

(30) Priority: 24.04.2009 US 172419 P
(43) Date of publication of application: 21.03.2012
(73) Proprietor: Corium International, Inc., Menlo Park, CA 94025 (US)
(72) Inventor: SAGI, Appala, Redwood City CA 94061 (US); TRAUTMAN, Joseph, C., Sunnyvale CA 94087 (US); CHEN, Guohua, Sunnyvale CA 94044 (US); WORSHAM, Robert, Wade, Cupertino CA 95014 (US); SINGH, Parminder, Union City CA 94587 (US)
(74) Representative: D Young & Co LLP
(86) International application number: PCT/US2010/032299
(87) International publication number: WO 2010/124255

(56) References cited:
- WO-A1-2005/082596
- WO-A2-2007/075806
- US-A1- 2002 193 819
- US-A1- 2003 195 474
- US-A1- 2004 164 454
- US-A1- 2005 178 760
- US-A1- 2007 191 761
- US-A1- 2008 269 685

## Description

### CROSS-REFERENCE TO RELATED APPLICATIONS

This application claims the benefit of U.S. Provisional Application No. 61/172,419, filed April 24, 2009.

### TECHNICAL FIELD

The subject matter described herein relates to generally to a process for manufacture of an array of microprojections for delivery of an active agent.

### BACKGROUND

Arrays of microneedles were initially proposed as a way of administering drugs through the skin in the 1970s, for example in expired U.S. Patent No. 3,964,482. Microneedle arrays can facilitate the passage of drugs through or into human skin and other biological membranes in circumstances where ordinary transdermal administration is inadequate. Microneedle arrays can also be used to sample fluids found in the vicinity of a biological membrane such as interstitial fluid, which is then tested for the presence of biomarkers.

In recent years it has become more feasible to manufacture microneedle arrays in a way that makes their widespread use financially feasible. U.S. Patent No. 6,451,240, for example, discloses some methods of manufacturing microneedle arrays. If the arrays are sufficiently inexpensive they may be marketed as disposable devices. A disposable device may be preferable to a reusable one in order to avoid the question of the integrity of the device being compromised by previous use and to avoid the potential need of resterilizing the device after each use and maintaining it in controlled storage.

Much of the initial work in fabricating microneedle arrays has focused on silicon or metals as the material of choice. There are significant advantages to using a polymer aw the material for manufacture of a polymeric arrays. For example, U.S. Patent No. 6,451,240 discloses some methods of manufacturing polymeric microneedle arrays. Arrays made primarily of biodegradable polymers have some advantages. U.S. Patent No. 6,945,952 and U.S. Published Patent Applications Nos. 2002/0082543, 2005/0197308 and 2008/0269685 describe microneedle arrays made of biodegradable polymers. A detailed description of the fabrication of a microneedle array made of polyglycolic acid is found in Jung-Hwan Park et al., "Biodegradable polymer microneedles: Fabrication, mechanics, and transdermal drug delivery," J. of Controlled Release, 104:51-66 (2005). U.S. Patent No. 7,578,954 discloses methods of constructing microstructures comprising through-holes. U.S. Patent Publication No. 2005/0178760 describes methods of making a microneedle array structure including hollow microneedles having a passageway extending therethrough. PCT Publication No. WO 2007/075806 describes a method making microstructures using injection molding. PCT Publication No. WO 2005/082596 describes a method of making a molded microneedle array using a negative mold insert in an injection molding apparatus.

Despite these efforts, there is still a need to find simpler and better methods for the manufacture of polymeric microneedle arrays and, in particular, microneedle arrays manufactured from biodegradable polymers. Desiderata include methods which waste a minimum of active ingredient and methods which place similar amounts of active in each individual microneedle of the array. A particular challenge to solvent casting microprojection arrays using a drug-polymer precursor has been the process of filling the mold with the precursor and avoiding air being trapped in the cavities of the mold which form the microprojections. If the air is not removed, the precursor may not fill the cavity to form the array or may take a long time to fill the cavity.

The foregoing examples of the related art and limitations related therewith are intended to be illustrative and not exclusive. Other limitations of the related art will become apparent to those of skill in the art upon a reading of the specification and a study of the drawings.

### BRIEF SUMMARY

In one aspect, a method of forming a microprojection array is provided. In the method, a substantially flexible mold is provided comprising a plurality of cavities that define individual microprojections in the array. A quantity of formulation is placed on a substrate, and the mold is placed in contact with the formulation so that the opening to each cavity in the plurality of cavities is in direct contact with the formulation. All or a portion of the formulation is transferred into the plurality of cavities. Then, either the mold is disengaged from the formulation, or the formulation is withdrawn from contact with the mold, in such a way that the formulation transferred into the plurality of cavities is retained.

In another aspect, a method of forming a microprojection array is provided. A mold is provided, the mold comprising a plurality of cavities for forming the microprojections. The mold is positioned in a fixture having first and second members, and at least one port. A formulation is introduced through the port into the fixture, for contact with openings to each of the cavities in the plurality of cavities in the mold. The formulation is transferred into the plurality of cavities, and then non-transferred formulation is withdrawn from contact with the mold at a rate whereby transferred formulation is retained in the plurality of cavities.

The method, in one embodiment, comprises dispensing a quantity of a formulation on a substrate, contacting a mold having a plurality of cavities for individual microprojections in the array with the formulation dispensed on the substrate, wherein the mold and the formulation are contacted such that the plurality of cavities are in fluid communication with the formulation, and then transferring formulation into the plurality of cavities. The mold is then disengaged from the formulation and the substrate in a manner that retains formulation in each of the plurality of cavities.

In one embodiment, transferring comprises transferring formulation into the plurality of cavities by applying pressure to the mold when in contact with the formulation dispensed on the substrate. In one embodiment, a pressure in the range of 0.1-1 atmosphere (10.1-101 kPa) is applied. In another embodiment, transferring comprises applying a vacuum to the mold when in contact with the formulation.

In another embodiment, the substrate has a reservoir or cavity. In yet another embodiment, the substrate comprises channels for venting a gas.

In one embodiment, disengaging the mold from the formulation and the substrate achieves a uniform retention of formulation in each of the plurality of cavities, as evidenced by a standard deviation in amount of formulation retained in each cavity less than or equal to about 10% of the average amount of formulation retained in the cavities.

In yet another embodiment, disengaging the mold from the formulation and the substrate is achieved by lifting an edge of the mold and gradually peeling the mold from the formulation and substrate at a controlled rate.

In one embodiment, the controlled rate of disengaging is carried out by a rotatable member attachable to the mold.

In still another embodiment, the method further comprises recovering formulation that is not retained in the plurality of cavities. In one embodiment at least about 90% of the formulation not retained in the plurality of the cavities is recovered.

The steps of dispensing, contacting, transferring and disengaging, individually or collectively, are conducted at a temperature of between about 23-25 °C.

In yet another embodiment, disengaging comprises disengaging at a rate of less than or equal to 5 mm/min.

The method further comprises, in some embodiments, removing solvent from the formulation that is retained in the plurality of cavities. In one embodiment, solvent is removed by placing the mold at a temperature to evaporate the solvent. In some embodiments, after removal of solvent, a second formulation is applied onto the mold.

The mold, in one embodiment, has a plurality of cavities that project into the mold from an approximately planar mold surface.

In another embodiment, for at least one cavity in the plurality of cavities, the diameter of the at least one cavity's intersection with a plane parallel to the planar mold surface decreases monotonically as a function of the plane's distance from the mold surface.

In still another embodiment, the diameter of the intersection of the at least one cavity with a plane parallel to the planar mold surface decreases more rapidly as when the plane is close to the mold surface than when the plane is further from the mold surface.

In yet another embodiment, the diameter of the intersection of the at least one cavity with a plane parallel to the planar mold surface decreases linearly as a function of distance to the mold surface for a range of distances close to the mold surface and then decreases linearly but more slowly for a second range of distances further away from the mold surface.

In another embodiment, a method of fabricating a microprojection array comprises positioning a mold comprising a plurality of cavities for forming microprojections in an array of microprojections in a fixture, the fixture comprised of a first member and a second member and at least one port, the mold positioned between the first and second members. A formulation is introduced into the fixture through the at least one port such that the formulation contacts openings to the plurality of cavities in the mold. All or a portion of the introduced formulation is then transferred into the plurality of cavities; and then any non-transferred formulation is withdrawn from the fixture at a rate whereby formulation transferred into the plurality of cavities is retained in each cavity in the plurality.

In one embodiment, transferring comprises pressurizing the formulation within the fixture.

In one embodiment, the steps of transferring and withdrawing achieve a uniform retention of formulation in each of the plurality of cavities, as evidenced by a standard deviation in amount of formulation retained in each cavity less than or equal to about 10% of the average amount of formulation retained in the cavities.

In yet another embodiment, a microprocessor controls the steps of introducing and withdrawing formulation from the fixture.

In another embodiment, the mold positioned in the fixture is a mold wherein the plurality of cavities are elongated cavities with a longer dimension and a shorter dimension, and the mold is held in the fixture in a way that the longer dimension of each cavity in the plurality of cavities is approximately horizontal.

The method, in another embodiment, further comprises removing the mold with retained formulation from the fixture.

In another embodiment, the method further comprises removing solvent from the retained formulation in each of the plurality of cavities to yield a dried formulation from which the microprojections it the array are composed.

In addition to the exemplary aspects and embodiments described above, further aspects and embodiments will become apparent by reference to the drawings and by study of the following descriptions.

### BRIEF DESCRIPTION OF THE DRAWINGS

FIGS. 1A-1B depict schematically in side-view two different shapes of a single microprojection from an array, where in FIG. 1A, the diameter of the microprojection decreases more rapidly with distance from the base closer to the base compared to further away from the base.
FIGS. 2A-2D depict schematically a method for filling a microprojection array mold with a formulation.
FIGS. 3A-3B are computer-generated photomicrographs of a microprojection array after filling with a formulation (FIG. 3A) and after drying of the formulation (FIG. 3B).
FIG. 4 is a computer-generated photomicrograph from an optical microscope, where the image is of microprojections in an array produced using the mold filling process described herein, wherein active agent that is concentrated in the tip of each microprojection is visible.
FIG. 5 schematically depicts an arrangement for peeling a mold according to one of the methods described herein.
FIGS. 6A-6B schematically depict a fixture for use in a method for filling microprojection cavities in a mold for a microneedle array with a liquid formulation that upon drying forms microprojections of the array.

### DETAILED DESCRIPTION

Before describing the methods of manufacture in detail, it is to be understood that the methods are not limited to specific solvents, materials, or device structures, as such may vary. It is also to be understood that the terminology used herein is for the purpose of describing particular embodiments only, and is not intended to be limiting.

As used in this specification and the appended claims, the singular forms "a," "an," and "the" include both singular and plural referents unless the context clearly dictates otherwise. Thus, for example, reference to "an active ingredient" includes a plurality of active ingredients as well as a single active ingredient, reference to "a temperature" includes a plurality of temperatures as well as single temperature, and the like.

For information regarding words which have multiple meanings, reference is made to The Oxford English Dictionary (2d ed. 1989), the McGraw-HilI Dictionary of Scientific and Technical Terms (6th ed. 2002) and to Hawley's Condensed Chemical Dictionary (15th ed. 2007). The inclusion of these references is not intended to imply that every definition in them is necessarily applicable here, as persons of skill in the art would often see that a particular definition is not in fact applicable in the present context.

Where a range of values is provided, it is intended that each intervening value between the upper and lower limit of that range and any other stated or intervening value in that stated range is encompassed within the disclosure. For example, if a range of 1 µm to 8 µm is stated, it is intended that 2 µm, 3 µm, 4 µm, 5 µm, 6 µm, and 7 µm are also disclosed, as well as the range of values greater than or equal to 1 µm and the range of values less than or equal to 8 µm.

The mold, in one embodiment, has a plurality of cavities that project into the mold from an approximately planar mold surface. In another embodiment, for at least one cavity in the plurality of cavities, the diameter of the at least one cavity's intersection with a plane parallel to the planar mold surface decreases monotonically as a function of the plane's distance from the mold surface. In still another embodiment, the diameter of the intersection of the at least one cavity with a plane parallel to the planar mold surface decreases more rapidly as when the plane is close to the mold surface than when the plane is further from the mold surface. In yet another embodiment, the diameter of the intersection of the at least one cavity with a plane parallel to the planar mold surface decreases linearly as a function of distance to the mold surface for a range of distances close to the mold surface and then decreases linearly but more slowly for a second range of distances further away from the mold surface.

### A. MICROPROJECTION ARRAYS

In this application reference is often made for convenience to "skin" as the biological membrane through which the active is administered. It will be understood by persons of skill in the art that in most or all instances the same inventive principles apply to administration through other biological membranes such as those which line the interior of the mouth, gastro-intestinal tract, blood-brain barrier, or other body tissues or organs or biological membranes which are exposed or accessible during surgery or during procedures such as laparoscopy or endoscopy.

In this application reference is also made to "microneedles" as a type of microprotrusion or microprojection which is being employed. It will be understood by persons of skill in the art that in many cases the same inventive principles apply to the use of other microprotrusions or microprojections to penetrate skin or other biological membranes. Other microprotrusions or microprojections may include, for example, microblades as described in U.S. Patent No. 6,219,574 and Canadian patent application no. 2,226,718, and edged microneedles as described in U.S. Patent No. 6,652,478.

In general it is preferred that the microprojections have a height of least about 50 µm, of at least about 100 µm, at least about 150 µm, at least about 200 µm, at least about 250 µm, or at least about 300 µm. In general it is also preferred that the microprojections have a height of no more than about 1 mm, no more than about 500 µm, no more than about 300 µm, or in some cases no more than about 200 µm or 150 µm. The microprojections may have an aspect ratio of at least 3:1 (height to diameter at base), at least about 2:1, or at least about 1:1. In one embodiment, a shape for the microprojections is a cone with a polygonal base, for example hexagonal or rhombus-shaped. Other possible microprojection shapes are shown, for example, in U.S. Published Patent App. 2004/0087992.

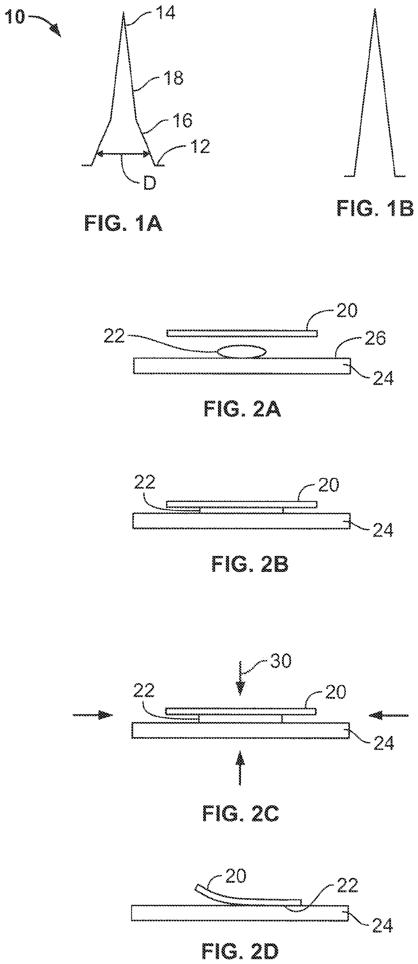

Microprojections may in some cases have a shape which becomes thicker towards the base, for example microprojections which have roughly the appearance of a funnel, or more generally where the diameter of the microprojection grows faster than linearly with increasing distance to the microprojection's distal end. Such a shape is beneficial to, for example, facilitate demolding. FIG. 1A schematically depicts in side-view an individual microprojection **10** of this type. As may be seen in the figure, the diameter D of the microprojection's intersection with a plane parallel to a base **12** decreases as the plane moves away from base **12** and closer to a tip **14** of the microprojection. In addition, this diameter decreases more rapidly close to base **12,** in basement zone **16,** than it does further away from the base, in tip zone **18.**

Where microprojections are thicker towards the base, a portion of the microprojection adjacent to the base, which is referred to herein as a "basement" region or a "foundation" region, approximately identified by zone **16** in FIG. 1A is designed not to penetrate the skin by virtue of its increasing diameter. As noted above, the microprojection of FIG. 1A is merely exemplary, and another example of a single microprojection in an array is illustrated in FIG. 1B.

The number of microprojections in an array is preferably at least about 100, at least about 500, at least about 1000, at least about 1400, at least about 1600, or at least about 2000. The area density of microprojections, given their small size, may not be particularly high, but for example the number of microprojections per cm² may be at least about 50, at least about 250, at least about 500, at least about 750, at least about 1000, or at least about 1500.

### B. Methods of Manufacture

In a first aspect, a method of forming a microprojection array, such as those described above, is provided. In one embodiment of the method and with reference to FIGS, 2A-2D, a microprojection array mold **20** is provided, the mold comprising a plurality of cavities that define individual microprotrusions or microprojections in the array. The microprojections are not visible in FIGS. 2A-2D due to their small size. Each cavity in the plurality of cavities has an opening and internal surface with dimensions, e.g, diameter and height, selected to provide a desired microprojection configuration. For example, the internal dimensions of the cavities have a certain height (or length) and diameter, which can vary along the height as evident from the exemplary microprojections shown in FIG. 1A-1B. The dimensions of the microprojection cavities in the mold correlate exactly or approximately, depending on various factors in the manufacturing process, to the dimensions of the finished microprojections in the array. A skilled artisan will appreciate that the cavities in the mold can all be the same or can differ, to yield a microprojection array with the same or differing microprojections.

Next, and with continuing reference to FIGS. 2A-2B, a quantity of a formulation **22,** described in more detail below, is placed on a substrate **24.** An optional release layer, such as a silicon sheet, can be placed on an upper surface **26** of the substrate to facilitate removal of the mold from the substrate. The substrate can include a recess or depression to retain the formulation (not shown in FIG. 2A). In substrate may also optionally comprises channels for venting a gas. The mold is placed in direct contact with formulation **22,** wherein the mold is oriented such that the opening to each cavity is in contact with the formulation. With respect to the drawing in FIG. 2A, the opening to each cavity is facing down. That is, the mold has an upper surface **28** whereupon the openings to each cavity in the plurality of cavities that define each microprojection in the array are disposed, and the upper surface is placed directly in contact with the formulation, such that the formulation can fill each cavity. Next, the formulation is moved or transferred into the cavities by a suitable means. In one embodiment, the suitable means is pressure, indicated in FIG. 2C by arrows, such as arrow 30. The mold, formulation, and substrate are placed, for example, in a pressure chamber, in order to transfer formulation into each cavity. A skilled artisan will appreciate alternatives to applying pressure to the mold are possible and contemplated. For example, a mold designed with a port on the surface opposing upper surface 28 or on a side wall can provide for attachment of a vacuum to achieve movement of the formulation into the plurality of cavities. Upon transport of the formulation into the cavities in the plurality, the mold is peeled away from the precursor formulation and the substrate in such a way that precursor is retained in the mold cavities, as depicted in FIG. 2D.

The mold may be manufactured by a wide variety of available techniques discussed in the literature. For example, it may be made by techniques discussed in U.S. Published Patent Application No. 2008/0269685. The mold may be made of synthetic polymeric materials having suitable mechanical properties. The mold should have mechanical properties such that the peeling step of the method can be carried out without difficulty and without damage to the mold. Thus, in one embodiment, the mold is fabricated from a flexible, polymeric material and in one embodiment is hydrophobic, and in another embodiment is permeable to oxygen, nitrogen and/or carbon dioxide.

The substrate may be flat on the side facing the mold. Alternatively, and as mentioned above, it may have a depression or cavity suitable for holding the microprojection precursor formulation. The substrate may be made, for example, of the same material of which the mold itself is made. The substrate may also be made of a polymer resin, such as polytetrafluoroethylene, polyethylene or polypropylene, or a metal, such as stainless steel, titanium or gold. In one embodiment, a material is placed in the depression which retains the formulation, particularly in the case where the mold and the substrate are hydrophobic in nature. A material placed in the cavity assists with distribution of the formulation in the depression and wetting of the interface between the substrate and the mold. An exemplary material is a non-woven material or non-woven netting fabric, such as a non-woven polyolefin available under the tradename DELNET^{®} (Delstar Technologies, Inc., Middletown, DE).

The placement of the precursor formulation may be done by dispensing, for example with a pipette, a single drop of precursor formulation on the substrate. Alternatively, more than one drop may be dispensed on the substrate, where the multiple drops can be arranged in a desirable pattern. If there is a depression in the substrate, the drop or drops may be placed in that depression.

The step of applying pressure to the mold, formulation, and substrate may be carried out, for example, by placing the components in a pressure chamber. Pressures greater than 0.2 atmospheres or greater than 0.5 atmospheres or greater than 1 atmosphere above atmospheric pressure may be employed.

A purpose of the step of applying pressure is to replace the air in the mold cavities with precursor formulation. There are other available techniques which help to achieve this objective, such as those set out in Published Patent Application No. 2008/0269685. It may be desirable to employ one or more of these alternative techniques and not carry out the step of applying pressure.

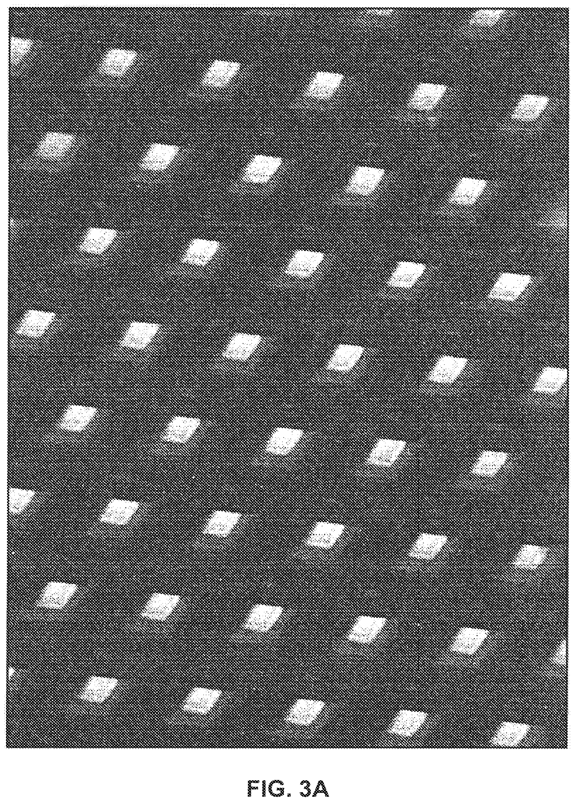

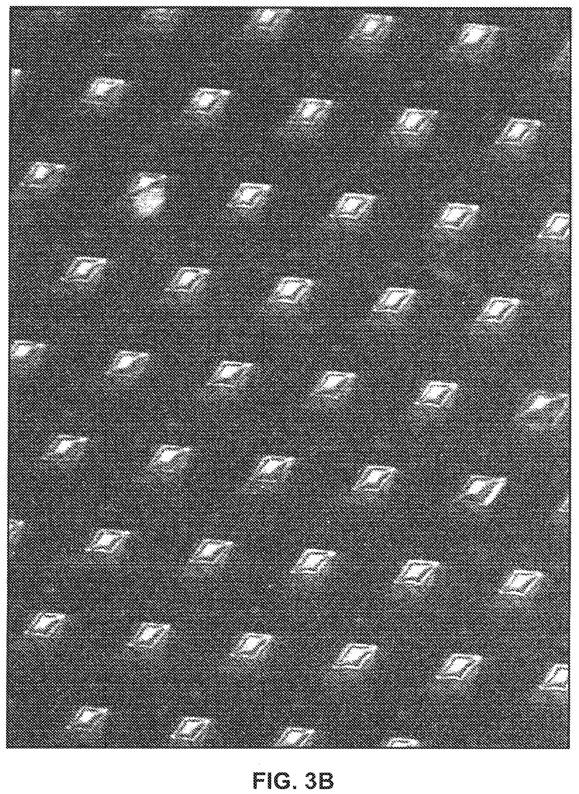

Example 3 below provides details on manufacture of microprojection arrays according to the processes described in FIGS. 2A-2D. In the working example, a metal substrate with a silicone release sheet was provided. A polymer and fluorescin formulation was applied to the silicone sheet, and the mold of the microprojection array was placed on the formulation, with the openings to each cavity in the mold in direct contact with the formulation. The substrate-silicone sheet-formulation-mold assembly was placed in a pressure chamber to transfer the formulation into the cavities of the mold. After removal of the pressure, the mold was removed from the assembly by lifting one edge of the mold and lifting so that the mold gradually disengages from the assembly. The microarrays prepared according to Example 3 were inspected optically, and the results are shown in FIGS. 3A-3B. In FIG. 3A, a photomicrograph of the upper surface of the microprojection array is seen, where the wet formulation is visible by virtue of the fluorescin dye. After the process of filling the microprojection cavities in the mold, the formulation is substantially or completely disposed within the cavities and essentially no formulation is on the surface of the mold between cavity openings. FIG. 3B shows the microarray after drying of the formulation and loss of solvent from the formulation, with the fluorescin dye, representative of an active drug, is deposited in the tip region of each microprojection.

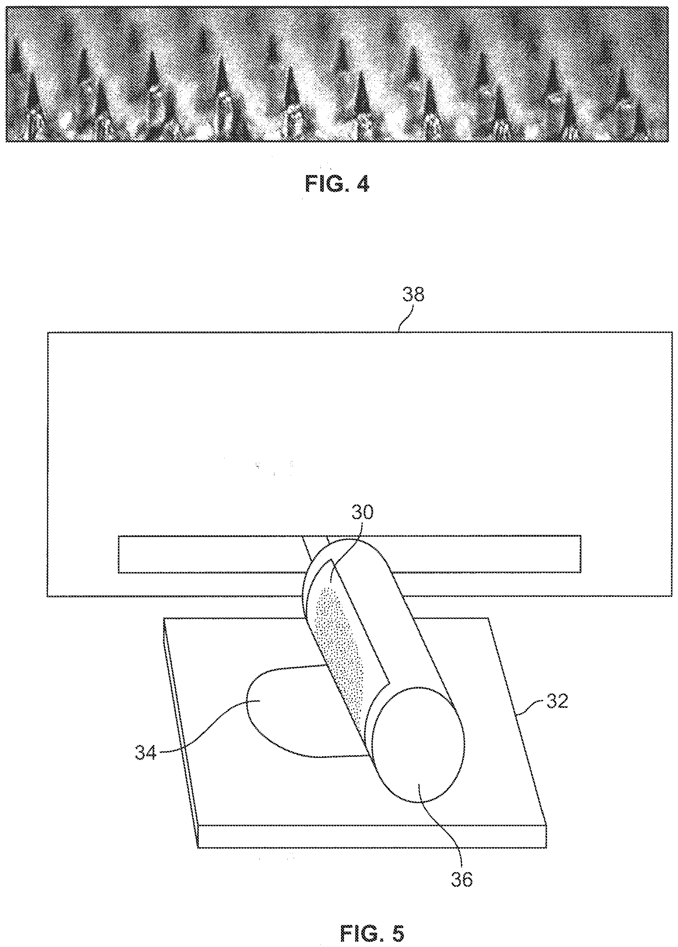

FIG. 4 is an artists rendering of an optical photomicrograph of microprojections in the array, the array fabricated from the process detailed in FIGS. 2A-2D. As seen, active agent is disposed primarily in the tip of each microprojection, and the larger diameter basement region of each microprojection (basement zone 16 in FIG. 1A) is essentially free of active agent.

A skilled artisan will appreciate that the orientation of the components in the process can be reversed, and the component that is removed, or 'peeled' away, changed. For example, a quantity of formulation can be placed on a mold which has the cavities facing up. A flexible substrate or sheet of material is placed atop the formulation. Pressure is applied to the sheet, precursor formulation, and mold. The substrate is peeled away or disengaged from the formulation and the mold in such a way that formulation is retained in the mold cavities.

Regardless of whether the mold or a substrate is peeled away, the peeling step may be accomplished, for example, manually with the fingers or more typically with an implement, such as tweezers. In carrying out the peeling manually, it may be desirable to watch the microprojection precursor formulation that is between the mold and the substrate and see that it is receding smoothly as the peeling operation ensues. It may also be desirable to attempt to maintain a more or less constant rate of peeling. The rate may be measured, for example, in millimeters per minute. Rates between about 1-10 mm/minute, about 3-7 mm/minute, or about 4-6 mm/minute may be employed. It may also be desirable to attempt to maintain a more or less constant angle between the mold as it is being peeled off and the substrate. It is preferred that the peel rate be controlled so that only microprojection formulation is left in the cavities of the mold. Preferably, none of the formulation should form a bridge between cavities or form droplets on the surface of the mold outside the cavities.

Alternatively, a mechanism may be employed to peel the mold away from the arrangement of components. For example, the mechanism may be a cylinder which is rolled over the substrate at a controllable speed, with the mold attached to the cylinder with the mold cavities facing outward. The mold may be attached, for example, with an adhesive. Alternatively, the attachment may take place by using a mechanical mode of attachment, for example protrusions that fit through holes in the mold or a mechanism that clips onto an end of the mold.

FIG. 5 depicts schematically a mechanism for peeling a mold **30** from a substrate **32** which has a shallow reservoir **34** in which a microprojection precursor formulation is retained. After dispensing a formulation into the reservoir, a microneedle mold is placed over the formulation-filled reservoir, so that the openings to each cavity in the mold is accessible by the formulation. All or a portion of the formulation is transferred into the cavities of the mold, for example, by placing the substrate, formulation and mold in a pressure vessel. Then, and with specific reference to FIG. 5 for this part of the process, the substrate, formulation, and mold are brought in contact with a cylinder **36.** The cylinder is rotatable by a mechanism **38** which may be based, for example, on a screw drive or on a stepper motor driving a pulley (not shown) or on any other manner of producing a linear motion of the cylinder parallel to the substrate, leaving the cylinder free to rotate. As cylinder **36** and mold **30** rotate with respect to each other, the mold detaches from substrate **32.** At suitable speeds and with suitable mold cavities and formulation material properties (e.g., viscosity, surface tension), the cavities in the mold that define each microprojection in the array retain formulation that, upon removal of the solvent from the formulation, yields a solid material in each cavity that is a microprojection. With respect to the means for transferring the formulation from the reservoir into the cavities of the mold, it will be appreciated that pressure is merely exemplary. Another approach is to apply a vacuum to the cylinder so that as the mold detaches from the substrate, formulation fluid is drawn into each cavity.

The mechanism **38** may operate under the control of an electric motor, which in turn may operate under the control of a computer or microprocessor. The use of electric motors to produce controlled motion over a short range is well known in the art, as is the computer control of electric motors. Reference may be made, for example, to H. Wayne Beaty & James L. Kirtley, Electric Motor Handbook (McGraw-Hill 1998). A skilled artisan will appreciate that it is alternatively possible to move the substrate while the cylinder remains stationary, to achieve movement of the mold away from the reservoir and its formulation.

When peeling the mold away from the formulation, the rate of peeling will have an influence on whether microprojection precursor formulation is retained in the mold cavities. A higher rate of peeling, *i.e,* a faster rate of removal of the mold from the formulation, tends to leave residual formulation droplets on the surface of the mold, whereas a lower rate of removal may pull all precursor formulation out of the cavities in the mold. Thus, in one embodiment, removal of the mold from contact with the formulation is selected to achieve the fastest removal rate with optimal and reliable retention of formulation in the mold cavities. One would thus, for example, try a variety of rates and see which ones produce reliable retention for that particular precursor and cavity geometry. Retention of formulation in the cavities of a mold may be determined, for example, by examining the cavities under a microscope to see if they appear partially filled, as depicted in FIG. 3B.

Without wishing to be bound by theory, it is believed that the process of retention of the microprojection precursor formulation in the mold cavities occurs because, as the mold is peeled away from the substrate, small drops of precursor break away from the mass of precursor formulation and remain in the cavities. One hypothesis is that the breakage of precursor occurs due to the local stress created by the peel. The stress is created by the opposing forces of liquid cohesion as it moves past the cavity and the liquid affinity to the cavity. The fact that this breakage occurs is unexpected, as is the fact that the process is reasonably reproducible and that the droplets that are retained in each of the cavities have approximately the same volume.

Example 4 describes another example of manufacture of a microprojection array according to the processes described in FIGS. 2A-2D. In this example, a microprojection array fabricated from a precursor formulation of dextran, sorbitol, the active agent human parathyroid hormone, in histidine buffer. After transfer of the formulation into the cavities of the mold, the mold was contacted with a moving member, such as a cylinder as depicted in FIG. 5. The cylinder was rotated to disengage the mold from the reservoir, where the step of disengaging the mold from the substrate was performed under conditions such that formulation transferred into the cavities of the mold was retained in the cavities, and the amount of formulation across the plurality of cavities was substantially uniform. In this example, the uniformity of formulation retained in the cavities of the mold was measured by quantifying the amount of active agent (human parathyroid hormone) in each microprojection. The coefficient of variation in the amount of active agent across the array of microprojections was 10%. In other embodiments, a coefficient of variation of less than 10%, more preferably of less than 7%, and still more preferably of less than about 5% or 3% is preferred.

As noted above, microprojections may in some cases have a shape which becomes thicker towards the base, for example microprojections which have roughly the appearance of a funnel, or more generally where the diameter of the microprojection grows faster than linearly with increasing distance to the microprojection's distal end. This shape of microprojection is depicted in FIG. 1A, where the basement region 16 is also referred to herein as a "funnel" due to its funnel-like shape. For molds designed to produce such roughly funnel-like microprojections, there may be a tendency for the thinner (distal) end of the funnel to retain microprojection precursor while the thicker (proximal) end of the funnel does not retain it.

It is generally desired that the amount of microprojection precursor formulation retained in the cavities be approximately the same from one cavity to the next. For example, it may be desired that the standard deviation of the volume of precursor formulation be less than about 30%, less than about 20%, less than about 10%, less than about 5%, or less than about 2% of the average volume of precursor formulation in the cavities which retain precursor formulation. An alternative manner of measuring the variation in the amount of precursor formulation from one cavity to the next in an array is in terms of the amount of active agent for the embodiments where the microprojection precursor formulation contains an active agent. For example, it may be desired that the standard deviation of the volumes or weights of active agent in the plurality of cavities in the microprojection array mold be less than about 30%, less than about 20%, less than about 10%, less than about 5%, or less than about 2% of the average volume or weight of active agent in the cavities which retain precursor formulation.

In another embodiment, another method of forming a microprojection array is provided. In this embodiment, one or more molds for a microneedle or microprojection array is secured in a fixture formed of two member, or halves, that mate in such a way to form a reservoir in which the one or more molds is disposed. A port is provided through the fixture and into the reservoir, through which a formulation material for formation of the microprojections in the array can be introduced. A second port or vent allows for passage of air that is displaced upon introduction of the formulation. The reservoir is filled with formulation in an amount sufficient to fill the plurality of cavities in the mold. The fixture is then exposed to a means that transfers the formulation from the reservoir into the pluralities in the mold, the means including pressure or vacuum or a combination of pressure and vacuum. Any formulation that is not transferred into the cavities in the mold is then controllably removed so that formulation transferred to the cavities is retained in the cavities, yet excess formulation is withdrawn. Desirably none of the formulation forms a bridge between cavities or forms droplets on the surface of the mold outside the cavities.

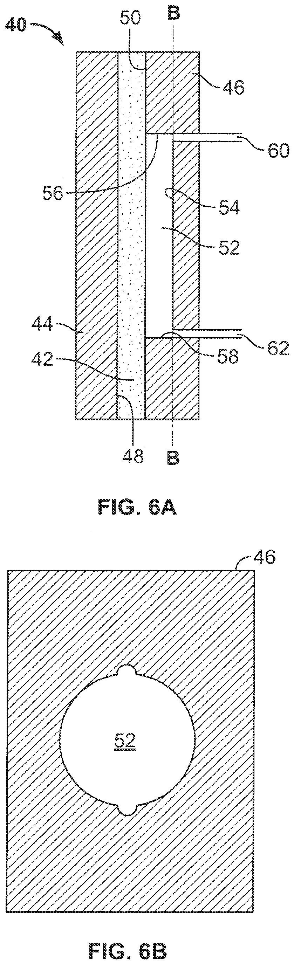

An exemplary apparatus suitable for practicing this embodiment is shown schematically in FIGS. 6A-6B. In the cross-sectional view of the fixture **40** shown in FIG. 6A, a mold **42** is held between first and second members, **44, 46,** of the fixture. First member 44 has an inner surface **48** and second member **46** has an inner surface **50.** One of the members, in this embodiment the second member, has a cavity **52** defined by a recessed floor **54** and sidewalls **56, 58.** First and second ports, **60, 62,** provide fluid communication between the external environment and cavity **52.** As will be described below, ports **60, 62** are used during the process for venting and pressurization with a gas, for introduction of formulation, and/or for evacuation of cavity (e.g., vacuum evacuation or pressurized evacuation). FIG. 6B is a cross-sectional top view of second member 46 taken along line B-B in FIG. 6A, that shows cavity **52** and ports **60, 62.**

In use, one or more microprojection molds which has openings to a plurality of cavities that define individual microprojections in the array is placed in the reservoir formed when the first and second members of the fixture are brought into contact. In FIG. 6A, a single mold 42 is secured adjacent cavity 52, where the openings to the plurality of cavities defining the microprojections of the array are in fluid communication with the cavity 52, also referred to as a reservoir when the two members are in a mating arrangement. In one embodiment, the reservoir has a depth of at least 0.05 mil (0.00127 mm), more preferably of at least 0.1 mil (0.00254 mm). The microprojection openings and the cavities are not shown in FIG. 6A due to their small size. Formulation is introduced through one of the ports, such as port **62,** and air or gas in the cavity is vented through the other port, such as port **60.** Enough formulation is introduced into the cavity to cover the plurality of openings to the cavities in the mold. Alternatively, it would be possible to leave some mold cavities uncovered, for example in order to produce a smaller array than the largest that a mold can produce. One of the ports is then closed, for example by suitable valving, and the reservoir is pressurized to a desired. A pressure gauge can be attached in member **46** to measure pressure in the cavity **52.** Pressurization of the reservoir can be achieved by introducing additional formulation into the reservoir after closing one of the ports or, alternatively, a pressurized gas may be introduced through one of the ports until the desired pressure in the reservoir is reached. The mold cavities desirably will remain covered with the formulation while the reservoir is being pressurized. Then, formulation that is not transferred into the cavities of the mold is withdrawn from the fixture reservoir in a manner that retains within the mold cavities the formulation that was transferred into the mold cavities. Formulation can be withdrawn by pressure in one port, vacuum in one port, or a combination of vacuum and pressure in the first and second ports.

The fixture may be provided with a mechanism which allows members **44, 46** to be separated and brought together using a handle or other convenient means of manipulation. The mechanism may be designed so that in a closed position the two members are pressed together. It may be desirable to place the microprojection array mold into the member with the cavity when the member is in a horizontal position, and then have the member which holds the mold movable into a vertical position for mating with the opposing member to form the fixture with the mold secured there between.

In another embodiment, the fixture is designed to contain more than one microprojection array mold, where the molds can be side-by-side with the openings to the cavities facing the reservoir or where the molds are situated in an opposing arrangement, so that the openings to the cavities face each other.

As with the method described above with respect to FIGS. 2A-2D, so too with the use of the method based on the fixture depicted in FIGS. 6A-6B it is believed that the process of retention of the microprojection precursor formulation in the mold cavities occurs because, as the level of precursor formulation falls, small drops of precursor formulation break away from the mass of precursor formulation and remain in the cavities. The fact that this breakage occurs is unexpected, as is the fact that the process is reproducible and that the droplets that are retained in the pluralities of cavities in the microprojection array mold have a uniform volume of formulation across the plurality of cavities, as measured by volume of formulation or by weight of active agent in each cavity. A "uniform" volume of formulation intends a standard deviation of the volumes or weights of active agent in the plurality of cavities in the microprojection array mold be less than about 30%, less than about 20%, less than about 10%, less than about 5%, or less than about 2% of the average volume of formulation or weight of active agent in the cavities which retain precursor formulation.

One variable which may affect under some circumstances whether microprojection precursor formulation remains in the cavities is the rate at which the formulation is removed from the reservoir, after the step of transferring the formulation into the cavities, for example by pressurization of the reservoir in the fixture. A controlled rate of withdrawal of formulation that remains in the reservoir after transfer of formulation into the plurality of cavities is selected, so that formulation that did not transfer into a cavity and remains in the reservoir is removed while retaining within each cavity the entire amount of formulation that was transferred into the microprojection cavity that will form the tip zone of each microprojection. A withdrawal rate of between about 0.1 cm/min and about 10 cm/min, or between about 0.2 cm/min and 2 cm/min, or between about 0.5 cm/min and 1.5 cm/min is generally suitable. The fill rate can be faster compared to the withdrawal rate.

Alternatively it may be convenient to measure or control withdrawal rate in mL/min rather than cm/min. As may be appreciated, where the mold is not rectangular, e.g., where it has a circular shape, the withdrawal rate in cm/min might not be proportional to the withdrawal rate in mL/min. For a circular reservoir, the withdrawal rate in mL/min may be varied, for example, by starting slowly (at the top of the reservoir), increasing until the level reaches the middle of the reservoir, and decreasing until the bottom of the reservoir is reached.

One would expect the desirable rates of precursor withdrawal for retention to depend at least somewhat on the viscosity and surface tension of the microprojection precursor and on its contact angle with the mold material. The desirable rates would also be expected to depend at least somewhat on the size and shape of the mold cavities and the shape and size of the reservoir. For example, quite large mold cavities might be unable to retain certain microprojection precursors at any withdrawal speed.

Examples 5-6 below detail two working example of the method of manufacture using a fixture as shown in FIGS. 6A-6B, where microprojection arrays were manufactured. With reference to Example 6, a microprojection array fabricated from a precursor formulation of dextran, sorbitol, the active agent human parathyroid hormone, in histidine buffer. A fixture dimensioned for retention of a single microprojection array mold in the fixture reservoir was used (referred to in Example 6 as a 'one-up' reservoir), and a fixture dimensioned for retention of two microprojection array molds in the fixture reservoir used (referred to in Example 6 as a 'two-up' reservoir). After placement of the one or more molds in the fixture, formulation was introduced into the reservoir via one of the ports, with the second port in an open position to vent air in the reservoir. Then, the second port is closed and the pressure in the reservoir is increased to transfer the formulation into the plurality of cavities in the mold(s). Formulation that remains in the reservoir after the transferring step is then removed from the reservoir at a rate that retains the transferred formulation in the cavities of the mold(s). The solvent in the formulation in the mold cavities was removed, by for example, placing the fixture containing the molds in an oven. The process was then repeated with a second formulation introduced into the reservoir of the fixture, to form a microprojection array wherein the basement region of each microprojection had a composition differing from the composition of the tip region.

Just as with peeling method of FIGS. 2A-2D, so with the use of a fixture as depicted in FIGS. 6A-6B, a mold with roughly funnel-shaped cavities can be used.

The mold may be manufactured by a wide variety of available techniques discussed in the literature. For example, it may be made by techniques discussed in U.S. Published Patent Application No. 2008/0269685. The mold may be made of synthetic polymeric materials having suitable mechanical properties. In general, molds may be used in this embodiment which are less flexible than those used in the peeling embodiment.
A skilled artisan will appreciate that removal of formulation from the fixture and/or introducing formulation into the fixture can be accomplished by any number of techniques, including but not limited to, manually or under the control of a microprocessor to ensure a uniform rate of fluid movement. In the working examples, a microprocessor-controlled syringe pump was used to introduce fluid into the fixture, and to remove fluid there from.

In either of the two embodiments discussed hereinabove, once the mold cavities have been filled, partially or completely with microprojection precursor formulation, they may be dried to remove solvent from the formulation, to yield a solid material in each cavity that forms the microprojections in the array. One or more additional layers of formulation may be applied over the first, preferably after it has dried, by the same or different method. Examples 1 and 2 together illustrate this approach. As a skilled artisan will appreciate, the additional formulation can have a different composition from the first, where the first formulation typically contains the active agent so that the agent is disposed in the tip of the microprojections, and the second formulation lacks active agent and forms the basement region of the microprojections that may not penetrate the stratum corneum.

A skilled artisan will appreciate that the various steps in the methods described herein, involving dispensing formulation, contacting formulation with the mold, transferring formulation into cavities of the mold, and disengaging the mold from the formulation or withdrawing formulation from a fixture, individually or collectively, can be conducted at a the same or different temperatures. In one embodiment all of the steps are conducted at room temperature, e.g., of between about 23-25 °C. recovering formulation that is not retained in the plurality of cavities.

A skilled artisan will appreciate that the methods described herein can include the step of recovering formulation that is not retained in the plurality of cavities in the mold. For example, after withdrawing formulation from the fixture, the formulation can be recovered for reuse or for analysis. For example, after disengaging the mold from the formulation deposited on the substrate, the formulation can be recovered for reuse or analysis. In one embodiment, at least about 90%, preferably 95%, of the formulation not retained in the plurality of the cavities is recovered.

In the methods described above, transfer of formulation into the plurality of cavities is achieved by a transfer step, which can be, for example, pressurizing the formulation, intending the formulation that is introduced into the reservoir of the fixture or the formulation that is deposited on a substrate (in which case the substrate-formulation and mold are generally pressurized together). A skill artisan can select a pressure that is suitable to achieve transfer of the formulation into the cavities of the mold, and will appreciate the variables that guide selection of the optimal pressure, including but not limited to volume, properties of the formulation, area, and temperature. In one embodiment, a pressure in the range of 0.1-1 atmosphere (10.1-101 kPa) is selected, and in preferred embodiments, a pressure of at least about 0.1 atm(10.1 kPa), at least about 0.5 atm (50.7 kPa), or at least about 1.0 atm (101 kPa) is applied.

### D. Formulations for the Microprojections and the Microprojection Array

A wide variety of formulations can be inserted into the mold cavities by the processes described herein. The formulation is referred to also as a "precursor formulation" to reflect that the formulation introduced into the mold cavities is a precursor of the resulting solid material from which the microprojections and/or microprojection array are made. Typically, the solvent of the precursor formulation is removed to yield the final material from which the microarray is made, however other changes to the precursor formulation can be designed to occur (such as crosslinking or reactions) or occur by happenstance.

In general the precursor formulations comprise at least one active agent (e.g. drug or therapeutic agent), at least one solvent, an optional polymer, and other optional ingredients such as sugars, antioxidants, preservatives, etc. Alternatively, the precursor formulations comprise at least one polymer, at least one solvent, an optional active agent, and other optional ingredients such as sugars, antioxidants, preservatives, etc. Optionally surfactants would be added to adjust surface tension. U.S. Published Patent Application No. 2008/0269685 discloses a wide variety of suitable materials for the precursor formulations.

The active therapeutic agents which may be placed in microprojections by the methods described herein include, but are not limited to, small molecule drugs, proteins, peptides, nucleic acids, and the like. In working Examples 5 and 6 below, human parathyroid hormone (hPTH) was used as an exemplary active agent. Vaccines are another exemplary therapeutic agent that can be included in the formulation for disperment in the microprojections of the array. U.S. Published Patent Application No. 2008/0269685 discloses a wide variety of suitable actives.

The microprojection precursor formulation may comprise one or more polymers. The polymers are preferably biocompatible. In another embodiment, the polymers are preferably biodegradable. By this term it is intended that a polymer will degrade under expected conditions of in vivo use (e.g., insertion into skin), irrespective of the mechanism of biodegradation. Exemplary mechanisms of biodegradation include disintegration, dispersion, dissolution, erosion, hydrolysis, and enzymatic degradation.

Exemplary polymers suitable for use in microprojection precursor formulations include, but are not limited to, poly(lactic acid), poly(glycolic acid), poly(lactic acid-coglycolic acid), poly(caprolactone), polyanhydrides, polyamines, polyesteramides, polyorthoesters, polydioxanones, polyacetals, polyketals, polycarbonates, polyphosphoesters, polyorthocarbonates, polyphosphazenes, poly(malic acid), poly(amino acids), hydroxycellulose, polyphosphoesters, dextran, tetrastarch, natural or modified polysaccharides, hyalouronidase, chitin, and copolymers, terpolymers and mixtures of these. In one embodiment, the formulation comprises dextran, and is preferably a dextran with a molecular weight of between about 20,000-100,000 Daltons, more preferably of between 40,000-80,000 Daltons, and still more preferably of between 40,000-70,000 Daltons. In specific examples, dextran with a molecular with of 70,000 Daltons (70 kDa) is used.

The microprojection precursor formulation may also comprise one or more sugars. Exemplary sugars which may be included in a microprojection array include dextrose, fructose, galactose, maltose, maltulose, iso-maltulose, mannose, lactose, lactulose, sucrose, and trehalose. Sugar alcohols, for example lactitol, maltitol, sorbitol, and mannitol, may also be employed. Cyclodextrins can also be used advantageously in microprojections arrays, for example α, β, and γ cyclodextrins, for example hydroxypropyl-β-cyclodextrin and methyl-β-cyclodextrin. Sugars and sugar alcohols may also be helpful in stabilization of certain actives (e.g., proteins) and in modifying the mechanical properties of the microprojections by a plasticizing-like effect. In one embodiment, sorbitol is a sugar included in the formulation, and in another embodiment, the formulation comprises sorbitol and a dextran.

The biodegradability of a microprojection array may be facilitated by inclusion of water-swellable polymers such as crosslinked PVP, sodium starch glycolate, celluloses, natural and synthetic gums, or alginates.

The microprojection arrays are suitable for a wide variety of drug substances. Suitable active agents that may be administered include the broad classes of compounds such as, by way of illustration and not limitation: analeptic agents; analgesic agents; antiarthritic agents; anticancer agents, including antineoplastic drugs; anticholinergics; anticonvulsants; antidepressants; antidiabetic agents; antidiarrheals; antihelminthics; antihistamines; antihyperlipidemic agents; antihypertensive agents; anti-infective agents such as antibiotics, antifungal agents, antiviral agents and bacteriostatic and bactericidal compounds; antiinflammatory agents; antimigraine preparations; antinauseants; antiparkinsonism drugs; antipruritics; antipsychotics; antipyretics; antispasmodics; antitubercular agents; antiulcer agents; anxiolytics; appetite suppressants; attention deficit disorder and attention deficit hyperactivity disorder drugs; cardiovascular preparations including calcium channel blockers, antianginal agents, central nervous system agents, beta-blockers and antiarrhythmic agents; caustic agents; central nervous system stimulants; cough and cold preparations, including decongestants; cytokines; diuretics; genetic materials; herbal remedies; hormonolytics; hypnotics; hypoglycemic agents; immunosuppressive agents; keratolytic agents; leukotriene inhibitors; mitotic inhibitors; muscle relaxants; narcotic antagonists; nicotine; nutritional agents, such as vitamins, essential amino acids and fatty acids; ophthalmic drugs such as antiglaucoma agents; pain relieving agents such as anesthetic agents; parasympatholytics; peptide drugs; proteolytic enzymes; psychostimulants; respiratory drugs, including antiasthmatic agents; sedatives; steroids, including progestogens, estrogens, corticosteroids, androgens and anabolic agents; smoking cessation agents; sympathomimetics; tissue-healing enhancing agents; tranquilizers; vasodilators including general coronary, peripheral and cerebral; vessicants; and combinations thereof.

Examples of peptides and proteins which may be used with microprojection arrays are oxytocin, vasopressin, adrenocorticotropic hormone (ACTH), epidermal growth factor (EGF), prolactin, luteinizing hormone, follicle stimulating hormone, luliberin or luteinizing hormone releasing hormone (LHRH), insulin, somatostatin, glucagon, interferon, gastrin, tetragastrin, pentagastrin, urogastrone, secretin, calcitonin, enkephalins, endorphins, kyotorphin, taftsin, thymopoietin, thymosin, thymostimulin, thymic humoral factor, serum thymic factor, tumor necrosis factor, colony stimulating factors, motilin, bombesin, dinorphin, neurotensin, cerulean, bradykinin, urokinase, kallikrein, substance P analogues and antagonists, angiotensin II, nerve growth factor, blood coagulation factors VII and IX, lysozyme chloride, renin, bradykinin, tyrocidin, gramicidines, growth hormones, melanocyte stimulating hormone, thyroid hormone releasing hormone, thyroid stimulating hormone, parathyroid hormone, pancreozymin, cholecystokinin, human placental lactogen, human chorionic gonadotropin, protein synthesis stimulating peptide, gastric inhibitory peptide, vasoactive intestinal peptide, platelet derived growth factor, growth hormone releasing factor, bone morphogenic protein, and synthetic analogues and modifications and pharmacologically active fragments thereof. Peptidyl drugs also include synthetic analogs of LHRH, e.g., buserelin, deslorelin, fertirelin, goserelin, histrelin, leuprolide (leuprorelin), lutrelin, nafarelin, tryptorelin, and pharmacologically active salts thereof.

Macromolecular active agents suitable for microprojection array administration may also include biomolecules such as antibodies, DNA, RNA, antisense oligonucleotides, ribosomes and enzyme cofactors such as biotin, oligonucleotides, plasmids, and polysaccharides. Oligonucleotides include DNA and RNA, other naturally occurring oligonucleotides, unnatural oligonucleotides, and any combinations and/or fragments thereof. Therapeutic antibodies include Orthoclone OKT3 (muromonab CD3), ReoPro (abciximab), Rituxan (rituximab), Zenapax (daclizumab), Remicade (infliximab), Simulect (basiliximab), Synagis (palivizumab), Herceptin (trastuzumab), Mylotarg (gemtuzumab ozogamicin), CroFab, DigiFab, Campath (alemtuzumab), and Zevalin (ibritumomab tiuxetan)..

Macromolecular active agents suitable for microprojection array administration may also include vaccines such as, for example, those approved in the United States for use against anthrax, diphtheria/tetanus/pertussis, hepatitis A, hepatitis B, *Haemophilus influenzae* type b, human papillomavirus, influenza, Japanese encephalitis, measles/mumps/rubella, meningococcal diseases (e.g., meningococcal polysaccharide vaccine and meningococcal conjugate vaccine), pneumococcal diseases (e.g., pneumococcal polysaccharide vaccine and meningococcal conjugate vaccine), polio, rabies, rotavirus, shingles, smallpox, tetanus/diphtheria, tetanus/diphtheria/pertussis, typhoid, varicelia, and yellow fever.

Because microprojection arrays penetrate human skin, it may be desirable to take steps which tend to eliminate the presence of microorganisms in the array. Such steps include, for example, the use of a formulation with high sugar concentration which will act as an osmotic agent to dehydrate microorganisms in the formulation. An alternative technique is the use of a non-physiological pH (e.g., below pH 6 and above pH 8) to retard growth and destroy microbial viability. The formulation may be made with organic solvents which are then dried in order to dehydrate microorganisms. Apart from the dehydration effect, the use of organic solvents is also inherently bactericidal since they disrupt bacterial cell membranes. In addition, the microprojection arrays may be packaged in a sealed, low oxygen environment to retard aerobic microorganisms and eventually destroy their viability. The arrays may also be packaged in a low moisture environment to dehydrate microorganisms.

A further technique to deal with microorganisms is to include a pharmaceutical acceptable antimicrobial agent in the formulation or the packaging. Examples of such agents are benzalkonium chloride, benzyl alcohol, chlorbutanol, meta cresol, esters of hydroxyl benzoic acid, phenol, and thimerosal.

As a further alternative, a surfactant or detergent can be added to the formulation to disrupt the cell membrane of any microorganisms to kill them. A desiccant could be added to the packaging to dehydrate microorganisms and kill them.

Antioxidants may be added to the formulation, for example to protect the active from oxidation. Exemplary antioxidants include methionine, cysteine, D-alpha tocopherol acetate, DL-alpha tocopherol, ascorbyl palmitate, ascorbic acid, butylated hydroxyanisole, butylated hydroxyquinone, butylhydroxyanisole, hydroxycomarin, butylated hydroxytoluene, cephalin, ethyl gallate, propyl gallate, octyl gallate, lauryl gallate, propylhydroxybenzoate, trihydroxybutyrophenone, dimethylphenol, ditertbutylphenol, vitamin E, lecithin, and ethanolamine.

It is to be understood that while the methods have been described in conjunction with the preferred specific embodiments thereof, the foregoing description is intended to illustrate and not limit the scope of the invention. Other aspects, advantages, and modifications within the scope of the invention will be apparent to those skilled in the art to which the invention pertains.

Where a patent, patent application, or publication containing express definitions is described herein, those express definitions should be understood to apply to the described patent, patent application, or publication in which they are found, and not to the remainder of the text of this application, in particular the claims of this application.

### EXAMPLES

The following examples are put forth so as to provide those of ordinary skill in the art with a complete disclosure and description of how to implement the methods described herein, and are not intended to limit the scope of what the inventors regard as their invention. Efforts have been made to ensure accuracy with respect to numbers (e.g., amounts, temperature, etc.) but some errors and deviations should be accounted for. Unless indicated otherwise, parts are parts by weight, temperature is in °C and pressure is at or near atmospheric.

### EXAMPLE 1

### GENERAL PROCESS FOR ARRAY CASTING

A microneedle mold is sterilized by e.g. dry heat or gamma irradiation. A formulation containing 25% bovine serum albumin (BSA), 20% polyvinyl alcohol, 27% trehalose, and 28% maltitol, with a total of 20% solids content in water, is prepared. An amount of the formulation (referred to herein as a precursor formulation), for example, 20 µL, is dispensed on the mold. The formulation is spread manually over the mold using a transfer pipette with a trimmed tip. The mold covered with formulation is then vortexed for five seconds using a commercial vibrating instrument to uniformly distribute the formulation across the mold. The mold with the formulation covering it is placed in a pressure vessel under 1 atm for about 10 minutes. Pressure is then removed. The mold is placed in an incubator at a temperature of 32°C, for about 30 minutes to 1 hour. The array is then demolded using double-sided adhesive tape, and is optionally attached to a backing.

### EXAMPLE 2

### GENERAL PROCESS FOR CASTING TWO-LAYER ARRAYS

Following the drying step and prior to demolding of Example 1, an additional layer is cast on the mold using a similar procedure. The additional layer consists of 75 µL of 20 wt% of the methacrylate cationic copolymer EUDRAGIT^{®} EPO (cationic copolymer based on dimethylaminoethyl methacrylate, butyl methacrylate, and methyl methacrylate) in a 3:1 mixture of ethanol and isopropyl alcohol. The additional layer is applied uniformly to the mold using a glass slide. The mold is placed in a pressure vessel and pressurized at 1 atm for 2 minutes. The pressure is released and the mold is allowed to dry in the pressure vessel for an additional five minutes, without disturbing. The mold is again dried in the incubator for 1 hour at 32°C, and then demolded.

### EXAMPLE 3

### PLACING MICROPROJECTION PRECURSOR IN MOLD CAVITIES USING PEELING

A microprojection array was manufactured as follows. A silicone sheet was placed on a flat metal substrate. 125 µL of a microprojection precursor formulation was dispensed as a drop on the silicone sheet using a micropipette. A flexible, polymeric microprojection array mold made of silicone was placed on the formulation with the plurality of openings to the plurality of cavities that define individual microprojections facing the formulation and the top surface of the mold in direct contact with the formulation. Each cavity in the plurality of cavities, wherein the cavity included both the tip region and the basement (or "funnel" region) had a volume of about 1 nL. The formulation was spread to cover a 1" x 1" (2.54 cm x 2.54 cm) area in the mold by manually applying pressure to the back side of the mold with tweezers. The assembly was placed in a pressure vessel and pressurized at 50 psi (344.7 kPa) for 1 minute. Pressure was released and the assembly was removed from the pressure vessel. Gripping one corner of the mold with tweezers, the mold was peeled from the silicone sheet.

The surface of the flexible mold conformed well with the surface of the silicone sheet. When the mold (after filling) was examined under a microscope (using UV light), the cavities appeared uniformly filled in a wet condition (FIG. 3A). No formulation was seen in the funnel area and filling was confined to the needle cavities only.

The silicone mold with formulation in the cavities was dried in an incubator at 32 °C for 30 minutes to 1 hour. As shown in FIG. 3B, the cavities continue to appear uniformly filled after drying. The mold was then placed on the mold carrier, alignment with a TEFLON^{®} wiper was checked, and the wiper clearance was set at 17 mils (0.043 cm) from the lowest point on the mold. 700 µL of a second formulation, a "basement" solution, was loaded in the array area and spread with a polyethyelene terephthalate (PET) cover slip. The mold with basement solution was pressurized at 50 psi (344.7 kPa) for 1 minute. Excess solution was wiped using the wiper, mounted perpendicular to the mold. The basement layer was dried under a hood at room temperature overnight, and then dried at 32°C in an incubator for a minimum of 15 minutes. This resulted in a microprojection array, which was optically inspected and a photomicrograph is shown in FIG. 4.

### EXAMPLE 4

### PLACING MICROPROJECTION PRECURSOR IN MOLD CAVITIES USING PEELING

A formulation comprised of 14% dextran 70, 4.8% sorbitol, 2.8% human parathyroid hormone (1 - 34) (hPTH) in histidine buffer solvent, pH 5.5, was prepared. The formulation was sterile filtered through a 0.22 micron filter. 250 µL of the formulation was dispensed into a shallow reservoir, a pre-sterilized silicone mold was placed on atop of shallow reservoir with the openings to each cavity of each microneedle in the mold downward facing so that the formulation can enter the cavities. The microprojection cavities in the mold were filled with the precursor formulation by pressurization of the substrate with the formulation-filled reservoir and the mold to 50 psi (344.7 kPa) in a pressure chamber for 1 minute. The mold was then attached to a cylinder and the cylinder was rotated with a speed of 2 - 4 mm/min to peel the mold from the shallow reservoir. The desirable amount of precursor formulation was retained in the cavities of the mold and dried at 32 °C for 30 minutes. Then a basement layer was coated on atop of dried precursor as described in Example 3, to form a microstructure array (die cut into 1 cm² in diameter). To analyze the hPTH content in each microprojection array, the array was optically inspected, and a photomicrograph is shown in FIG. 4. The drug concentrated in the tips of the microprojections is observed. Then the microarray was dissolved in buffer and hPTH content was measured by HPLC. With a sample size of 30 arrays tested, the average hPTH content per array was 32.9 µg with the coefficient of variation of 10%.

### EXAMPLE 5

### PLACING MICROPROJECTION PRECURSOR IN MOLD USING FIXTURE

A 1 inch by 1 inch (2.54 cm x 2.54 cm) mold for a microprojection array was provided. Each cavity in the plurality of cavities in the mold, wherein the cavity included both the tip region and the basement (or "funnel" region), had a volume of about 1 nL. A microprojection precursor formulation comprised of 2.1% hPTH (1-34), 14% tetrastarch, 4.8% sorbitol in histidine buffer solvent, pH 5.5 was prepared.

A fixture comprised of first and second members was provided (FIG. 6A). The second member comprised a cylindrical cavity (referred to as the reservoir) with a diameter of 22 mm and a depth of 20 mils (0.508 mm), giving a volume of approximately 200 µL. The members of the fixture were made of metal coated with polytetrafluoroethylene (PTFE).

The neck of a 1 mL sterile syringe was dipped into the formulation and about 1 mL drawn into the barrel. Air trapped inside the barrel near the plunger head was removed with gentle taps to the syringe. A tubing was attached to an inlet port on the fixture and the opposing end of the tubing attached to the syringe neck. The line was purged to ensure no air bubbles were in the tubing.

The mold was loaded into the fixture, and clamped shut. The syringe with formulation was placed on a syringe pump, which was then turned on, and formulation was introduced into the reservoir of the fixture at 0.95 mL/minute. After filling, the reservoir formulation flows into the venting inlet. When formulation was filled about 2 inches (5.1 cm) into the venting inlet tubing, the syringe pump was stopped.

A valve was employed to close off the venting inlet tube. The pressure in the reservoir was then gradually increased to 50 psi (344.7 kPa). During the pressure increase process, while the formulation occupies the mold cavity to replace the air in the cavity the level of formulation in the venting inlet tube will fall somewhat, so it may be necessary to pump additional formulation to maintain the level of formulation in that tube at about 2 inches (5.1 cm) during the course of pressurization. When 50 psi (344.7 kPa) was reached, the pressure was held for 1 minute and was then released gradually.

The step of withdrawing microprojection precursor formulation began. This step extended over a period of 6-10 minutes, so the average rate of withdrawal of roughly 200 µL of formulation was about 20-30 µL/min. The formulation was withdrawn at an initial rate of roughly 15 µL/min, rising towards a higher rate as the level of formulation reached the center of the mold and then again at a slower rate as the level of formulation continued to fall. When liquid formulation withdrawal from the reservoir was complete, the formulation moved into the inlet tubing. The pump was stopped, the fixture was opened, and the mold removed and placed in a Petri dish. The mold was examined for uniformity of fill and specks of microprojection precursor around the periphery. After drying the mold in an incubator at 32°C for 30 minutes, the Petri dish cover was closed, the dish was placed in a foil pouch and heat sealed with nitrogen fill, and was then stored in a refrigerator at 4°C.

A further layer (25% poly(lactic-co-glycolic acid) in acetonitrile) was cast over the formulation in the mold cavities and dried overnight. The resulting array was demolded using a PET strip with 1513 double coated adhesive. The array was notch cut with diameter 11 mm, labeled, and stored in a dry chamber.

Active content and purity were determined using HPLC. The standard deviation of active content in the mold cavities was ∼ 6%.

### EXAMPLE 6

### PLACING MICROPROJECTION PRECURSOR IN MOLD CAVITIES USING PEELING

A mold for a microprojection array, 18 mm by 30 mm, was obtained. A precursor comprising of 14% Dextran 70, 4.8% sorbitol, 2.1% human parathyroid hormone (1 - 34) (hPTH) in histidine buffer solvent, pH 5.5, was prepared. The precursor formulation was sterile filtered through a 0.22 µm filter.

Fixtures comprised of first and second members was provided (FIG. 6A). In one fixture, the second member comprised a cylindrical cavity (or reservoir) with diameter 22 mm and depth 53 mils (1.35 mm, referred to herein as a "one-up reservoir"). In a second fixture, the second member comprised a rectangular reservoir with a length of 28 mm and a width of 16 mm referred to herein as a "two-up reservoir"). The fixture with the two-up reservoir can be oriented either vertically or horizontally in use.

The neck of a 2 mL syringe (sterile by autoclave) was dipped into precursor and about 1.5 mL of precursor formulation was drawn into the barrel. Gentle taps removed any air trapped in the syringe. An inlet tubing was attached to the syringe neck and the line was purged to ensure no air bubbles in the tubing.

The mold was loaded into the fixture, and clamped shut. The syringe with formulation was placed on a syringe pump. The syringe pump was started and the formulation was introduced into the cavity of the fixture at a rate of 0.95 mL/min. After filling, the reservoir precursor formulation flowed into the venting inlet. When precursor formulation is filled about 2 inch (5.1 cm) into the venting inlet tubing, the syringe pump was stopped.

A valve was employed to close off the venting inlet tube. The pressure in the reservoir was then gradually increased to 50 psi (344.7 kPa), as described in Example 5. After pressurization, the precursor formulation was withdrawn from reservoir at a certain speed. A desirable amount of precursor formulation was retained in the cavities of the mold(s) after completion of withdrawing of precursor formulation from the reservoir. The precursor formulation in cavities of mold was dried at 32 °C for 30 min. Then a basement layer was coated on atop of dried precursor formulation as described in the examples above, to form microstructure array (die cut into 1 cm² in diameter).

To analyze the hPTH content in each microprojection array, the array was dissolved in buffer and hPTH content was measured by HPLC. As shown in the table below, the amount of precursor formulation retained in the microprojection cavities per cm² array was affected by several factors including, but not limited to, reservoir orientation, depth, precursor withdrawing rate, etc.

| Group | Reservoir type | Orientation | Reservoir depth mil (mm) | Withdraw rate (mm/min) | hPTH content (µg/cm² array) |
|---|---|---|---|---|---|
| 1 | Cylindrical (one-up) | NA | 52 (1.3) | 12 | 21.6 ± 2.8 |
| 2 | Rectangle (two-up) | Vertical | 35 (0.89) | 3 | 52.1 ± 5.9 |
| 3 | Rectangle (two-up) | Vertical | 35 (0.89) | 12 | 86.1 ± 9.2 |
| 4 | Rectangle (two-up) | Vertical | 49 (1.24) | 7.5 | 74.7 ± 9.8 |
| 5 | Rectangle (two-up) | Vertical | 63 (1.6) | 3 | 45.3 ± 2.8 |
| 6 | Rectangle (two-up) | Vertical | 63 (1.6) | 12 | 66.5 ± 4.1 |
| 7 | Rectangle (two-up) | Horizontal | 63 (1.6) | 3 | 18.8 ± 2.7 |

While a number of exemplary aspects and embodiments have been discussed above, those of skill in the art will recognize certain modifications, permutations, additions and sub-combinations thereof. It is therefore intended that the following appended claims and claims hereafter introduced are interpreted to include all such modifications, permutations, additions and sub-combinations.

## Claims

1. A method of forming a microprojection array, comprising:
dispensing a quantity of a formulation (22) on a mold (20) having a plurality of cavities for individual microprojections in the array , wherein the mold and the formulation are contacted such that the plurality of cavities are in fluid communication with the formulation;
covering the formulation on the mold with a substrate;
transferring the formulation into the plurality of cavities; and
removing excess formulation from the mold in a manner that retains formulation in each of the plurality of cavities to form the microprojection array.

2. The method according to claim 1, wherein removing excess formulation from the mold includes disengaging the mold from the substrate.

3. A method of fabricating a microprojection array, comprising:
positioning a mold comprising a plurality of cavities for forming microprojections in an array of microprojections in a fixture (40), the fixture comprised of a first member (44) and a second member (46) and at least one port (60, 62), the mold positioned between the first and second members;
introducing a formulation into the fixture through the at least one port such that the formulation contacts openings to the plurality of cavities in the mold;
transferring the formulation introduced into the fixture into the plurality of cavities; and
withdrawing formulation from the fixture at a rate whereby formulation transferred into the plurality of cavities is retained in each cavity in the plurality to form the microprojection array.

4. The method according to claim 1, wherein transferring comprises transferring formulation into the plurality of cavities by applying pressure to the formulation dispensed on the mold.

5. The method according to claim 1 or 2, wherein removing excess formulation from the mold achieves a uniform retention of formulation in each of the plurality of cavities, as evidenced by a standard deviation in amount of formulation retained in each cavity less than or equal to about 10% of the average amount of formulation retained in the cavities.

6. The method according to claim 2 or claim 5, wherein removing excess formulation from the mold is achieved by lifting an edge' of the mold and gradually peeling the mold from the formulation and substrate at a controlled rate.

7. The method according to claim 6, wherein the controlled rate of disengaging is carried out by a rotatable member (36) attachable to the mold.

8. The method according to claim 3, wherein the steps of transferring and withdrawing achieve a uniform retention of formulation in each of the plurality of cavities, as evidenced by a standard deviation in amount of formulation retained in each cavity less than or equal to about 10% of the average amount of formulation retained in the cavities.

9. The method according to any one of claims 1 to 2 or claim 3, further comprising recovering formulation that is not retained in the plurality of cavities.

10. The method according to claim 9, wherein at least about 90% of the formulation not retained in the plurality of the cavities is recovered.

11. The method according to any preceding claim, wherein one or more of the steps in the method is conducted at a temperature of between about 23-25 °C.

12. The method according to any preceding claim, wherein the formulation comprises at least one polymer dissolved in a solvent, the method further comprising removing solvent from the formulation retained in the plurality of cavities.

13. The method according to any preceding claim, further comprising applying a second formulation onto the mold.

14. The method according to any preceding claim, wherein the plurality of cavities project into the mold from an approximately planar mold surface.

15. The method according to claim 14, wherein for at least one cavity in the plurality of cavities, the diameter of the at least one cavity's intersection with a plane parallel to the planar mold surface decreases monotonically as a function of the plane's distance from the mold surface.

16. The method according to claim 15, wherein the diameter of the intersection of the at least one cavity with a plane parallel to the planar mold surface decreases more rapidly as when the plane is close to the mold surface than when the plane is further from the mold surface.

17. The method according to claim 16, wherein the diameter of the intersection of the at least one cavity with a plane parallel to the planar mold surface decreases linearly as a function of distance to the mold surface for a range of distances close to the mold surface and then decreases linearly but more slowly for a second range of distances further away from the mold surface.

18. The method according to claim 3 or 8, wherein positioning includes positioning a mold wherein the plurality of cavities are elongated cavities with a longer dimension and a shorter dimension, and the mold is held in the fixture in a way that the longer dimension of each cavity in the plurality of cavities is approximately horizontal.

19. The method according to any one of claims 3, 8 or 18, wherein the introducing and withdrawing formulation steps are conducted under the control of a microprocessor.

20. The method according to any one of claims 3, 8, 18 or 19, further comprising removing the mold with retained formulation from the fixture.

21. The method according to any preceding claim, wherein the formulation comprises at least one polymer dissolved in a solvent, the method further comprising removing solvent from the retained formulation in each of the plurality of cavities.

## Patentansprüche

1. Verfahren zur Bildung eines Mikroprojektionsarrays, umfassend:
Abgeben einer Menge einer Formulierung (22) auf eine Form (20) mit einer Vielzahl von Hohlräumen für individuelle Mikroprojektionen in dem Array, wobei die Form und die Formulierung so in Kontakt kommen, dass die Vielzahl der Hohlräume in Fluidverbindung mit der Formulierung ist;
Bedecken der Formulierung auf der Form mit einem Substrat;
Transferieren der Formulierung in die Vielzahl der Hohlräume; und
Entfernen von überschüssiger Formulierung aus der Form in einer Weise, die Formulierung in jeder der Vielzahl der Hohlräume zurückhält, um das Mikroprojektionsarray zu bilden.

2. Verfahren nach Anspruch 1, wobei das Entfernen von überschüssiger Formulierung aus der Form das Trennen der Form von dem Substrat einschließt.

3. Verfahren zur Fertigung eines Mikroprojektionsarrays, umfassend:
Positionieren einer Form, die eine Vielzahl der Hohlräume zur Bildung von Mikroprojektionen in einem Array von Mikroprojektionen umfasst, in einer Halteeinrichtung (40), wobei die Halteeinrichtung ein erstes Element (44) und ein zweites Element (46) und mindestens einen Anschluss (60, 62) dazwischen umfasst, wobei die Form zwischen dem ersten und dem zweiten Element positioniert ist;
Einbringen einer Formulierung in die Halteeinrichtung durch den mindestens einen Anschluss hindurch, so dass die Formulierung in Kontakt mit Öffnungen der Vielzahl der Hohlräume in der Form kommt;
Transferieren der in die Halteeinrichtung eingebrachten Formulierung in die Vielzahl der Hohlräume; und
Entnehmen von Formulierung aus der Halteeinrichtung mit einer Rate, wobei in die Vielzahl der Hohlräume transferierte Formulierung in jedem Hohlraum in der Vielzahl zurückgehalten wird, um das Mikroprojektionsarray zu bilden.

4. Verfahren nach Anspruch 1, wobei das Transferieren das Transferieren von Formulierung in die Vielzahl der Hohlräume umfasst, indem Druck auf die Formulierung ausgeübt wird, die auf die Form abgegeben wird.

5. Verfahren nach Anspruch 1 oder 2, wobei das Entfernen von überschüssiger Formulierung aus der Form ein gleichförmiges Zurückhalten von Formulierung in jedem von der Vielzahl der Hohlräume erreicht, wie durch eine Standardabweichung in der Menge der Formulierung, die in jedem Hohlraum zurückgehalten wird, von kleiner als oder gleich etwa 10 % der durchschnittlichen Menge der Formulierung deutlich wird, die in den Hohlräumen zurückgehalten wird.

6. Verfahren nach Anspruch 2 oder Anspruch 5, wobei das Entfernen von überschüssiger Formulierung aus der Form erreicht wird, indem ein Rand der Form angehoben wird und allmählich die Form von der Formulierung und dem Substrat mit einer kontrollierten Rate abgezogen wird.

7. Verfahren nach Anspruch 6, wobei die kontrollierte Rate des Trennens mit einem drehbaren Element (36) durchgeführt wird, das an der Form befestigbar ist.

8. Verfahren nach Anspruch 3, wobei die Schritte des Transferierens und Entnehmens ein gleichförmiges Zurückhalten von Formulierung in jedem von der Vielzahl der Hohlräume erreichen, wie durch eine Standardabweichung in der Menge der Formulierung, die in jedem Hohlraum zurückgehalten wird, von kleiner als oder gleich etwa 10 % der durchschnittlichen Menge der Formulierung deutlich wird, die in den Hohlräumen zurückgehalten wird.

9. Verfahren nach einem der Ansprüche 1 bis 2 oder Anspruch 3, ferner umfassend Gewinnen von Formulierung, die nicht in der Vielzahl der Hohlräume zurückgehalten wird.

10. Verfahren nach Anspruch 9, wobei mindestens etwa 90 % der Formulierung, die nicht in der Vielzahl der Hohlräume zurückgehalten wird, gewonnen werden.

11. Verfahren nach einem der vorhergehenden Ansprüche, wobei ein oder mehrere der Schritte in dem Verfahren bei einer Temperatur zwischen etwa 23 und 25 °C durchgeführt werden.

12. Verfahren nach einem der vorhergehenden Ansprüche, wobei die Formulierung mindestens ein in einem Lösungsmittel gelöstes Polymer umfasst, wobei das Verfahren ferner das Entfernen von Lösungsmittel aus der Formulierung umfasst, die in der Vielzahl der Hohlräume zurückgehalten wurde.

13. Verfahren nach einem der vorhergehenden Ansprüche, ferner umfassend Aufbringen einer zweiten Formulierung auf die Form.

14. Verfahren nach einem der vorhergehenden Ansprüche, wobei die Vielzahl der Hohlräume von einer ungefähr ebenen Formoberfläche in die Form ragt.

15. Verfahren nach Anspruch 14, wobei für mindestens einen Hohlraum in der Vielzahl der Hohlräume der Durchmesser des Schnittpunkts des mindestens einen Hohlraums mit einer Ebene, die parallel zu der ebenen Formoberfläche verläuft, als Funktion des Abstands der Ebene von der Formoberfläche monoton abnimmt.

16. Verfahren nach Anspruch 15, wobei der Durchmesser des Schnittpunkts des mindestens einen Hohlraums mit einer Ebene, die parallel zu der ebenen Formoberfläche verläuft, rascher abnimmt, wenn die Ebene nahe an der Formoberfläche ist, als wenn die Ebene von der Formoberfläche weiter entfernt ist.

17. Verfahren nach Anspruch 16, wobei der Durchmesser des Schnittpunkts des mindestens einen Hohlraums mit einer Ebene parallel zu der ebenen Formoberfläche für einen Bereich von Abständen nahe an der Formoberfläche als Funktion des Abstands zu der Formoberfläche linear abnimmt und dann für einen zweiten Bereich von Abständen, die von der Formoberfläche weiter entfernt sind, linear, jedoch langsamer abnimmt.

18. Verfahren nach Anspruch 3 oder 8, wobei das Positionieren das Positionieren einer Form einschließt, wobei die Vielzahl der Hohlräume längliche Hohlräume mit einer längeren Abmessung und einer kürzeren Abmessung sind, und die Form in einer Weise in der Halteeinrichtung gehalten wird, dass die längere Abmessung jedes Hohlraums in der Vielzahl der Hohlräume annähernd horizontal ist.

19. Verfahren nach einem der Ansprüche 3, 8 oder 18, wobei die Schritte des Einbringens und Entnehmens der Formulierung unter Steuerung eines Mikroprozessors durchgeführt werden.

20. Verfahren nach einem der Ansprüche 3, 8, 18 oder 19, ferner umfassend Entfernen der Form mit zurückgehaltener Formulierung aus der Halteeinrichtung.

21. Verfahren nach einem der vorhergehenden Ansprüche, wobei die Formulierung mindestens ein in einem Lösungsmittel gelöstes Polymer umfasst, wobei das Verfahren ferner das Entfernen von Lösungsmittel aus der zurückgehaltenen Formulierung in der Vielzahl der Hohlräume umfasst.

## Revendications

1. Procédé de formation d'un réseau de microsaillies, comprenant :
la distribution d'une quantité d'une formulation (22) sur un moule (20) ayant une pluralité de cavités pour microsaillies individuelles dans le réseau, le moule et la formulation étant mis en contact de telle sorte que la pluralité de cavités sont en communication fluidique avec la formulation ;
le recouvrement de la formulation sur le moule avec un substrat ;
le transfert de la formulation dans la pluralité de cavités ; et
le retrait de l'excès de formulation du moule d'une manière qui retient la formulation dans chacune de la pluralité de cavités pour former le réseau de microsaillies.

2. Procédé selon la revendication 1, dans lequel le retrait de l'excès de formulation du moule comporte la séparation du moule du substrat.

3. Procédé de fabrication d'un réseau de microsaillies, comprenant :
le positionnement d'un moule comprenant une pluralité de cavités pour former des microsaillies dans un réseau de microsaillies dans un montage (40), le montage composé d'un premier élément (44) et d'un deuxième élément (46) et d'au moins un orifice (60, 62), le moule positionné entre le premier et le deuxième élément ;
l'introduction d'une formulation dans le montage par l'au moins un orifice de telle sorte que la formulation entre en contact avec les ouvertures de la pluralité de cavités dans le moule ;
le transfert de la formulation introduite dans le montage dans la pluralité de cavités ; et
le retrait de la formulation du montage à une vitesse à laquelle la formulation transférée dans la pluralité de cavités est retenue dans chaque cavité dans la pluralité pour former le réseau de microsaillies.

4. Procédé selon la revendication 1, dans lequel le transfert comprend le transfert de la formulation dans la pluralité de cavités par application d'une pression à la formulation distribuée sur le moule.

5. Procédé selon la revendication 1 ou 2, dans lequel le retrait de l'excès de formulation du moule permet d'obtenir une rétention uniforme de formulation dans chacune de la pluralité de cavités, comme en témoigne un écart type de la quantité de formulation retenue dans chaque cavité inférieur ou égal à environ 10 % de la quantité moyenne de formulation retenue dans les cavités.

6. Procédé selon la revendication 2 ou la revendication 5, dans lequel le retrait de l'excès de formulation du moule est réalisé en levant un bord du moule et en décollant progressivement le moule de la formulation et du substrat à une vitesse contrôlée.

7. Procédé selon la revendication 6, dans lequel la vitesse contrôlée de séparation est imprimée par un élément rotatif (36) fixable au moule.

8. Procédé selon la revendication 3, dans lequel les étapes de transfert et de retrait permettent d'obtenir une rétention uniforme de formulation dans chacune de la pluralité de cavités, comme en témoigne un écart type de la quantité de formulation retenue dans chaque cavité inférieur ou égal à environ 10 % de la quantité moyenne de formulation retenue dans les cavités.

9. Procédé selon l'une quelconque des revendications 1 à 2 ou la revendication 3, comprenant en outre la récupération de la formulation qui n'est pas retenue dans la pluralité de cavités.

10. Procédé selon la revendication 9, dans lequel au moins 90 % environ de la formulation non retenue dans la pluralité des cavités est récupérée.

11. Procédé selon une quelconque revendication précédente, une ou plusieurs des étapes du procédé étant conduites à une température comprise entre environ 23 et 25 °C.

12. Procédé selon une quelconque revendication précédente, dans lequel la formulation comprend au moins un polymère dissous dans un solvant, le procédé comprenant en outre le retrait du solvant de la formulation retenue dans la pluralité de cavités.

13. Procédé selon une quelconque revendication précédente, comprenant en outre l'application d'une deuxième formulation sur le moule.

14. Procédé selon une quelconque revendication précédente, dans lequel la pluralité de cavités font saillie dans le moule depuis une surface du moule approximativement plane.

15. Procédé selon la revendication 14, dans lequel pour au moins une cavité dans la pluralité de cavités, le diamètre de l'intersection de l'au moins une cavité avec un plan parallèle à la surface plane du moule diminue de façon monotone en fonction de la distance du plan à la surface du moule.

16. Procédé selon la revendication 15, dans lequel le diamètre de l'intersection de l'au moins une cavité avec un plan parallèle à la surface plane du moule diminue plus rapidement lorsque le plan est proche de la surface du moule que lorsque le plan est plus éloigné de la surface du moule.

17. Procédé selon la revendication 16, dans lequel le diamètre de l'intersection de l'au moins une cavité avec un plan parallèle à la surface plane du moule diminue linéairement en fonction de la distance à la surface du moule pour une gamme de distances proches de la surface du moule et diminue ensuite linéairement, mais plus lentement pour une deuxième gamme de distances plus éloignées de la surface du moule.

18. Procédé selon la revendication 3 ou 8, dans lequel le positionnement comporte le positionnement d'un moule dans lequel la pluralité de cavités sont des cavités allongées avec une dimension plus longue et une dimension plus courte, et le moule est maintenu dans le montage de telle sorte que la dimension plus longue de chaque cavité dans la pluralité de cavités est approximativement horizontale.

19. Procédé selon l'une quelconque des revendications 3, 8 ou 18, dans lequel les étapes d'introduction et de retrait de la formulation sont conduites sous le contrôle d'un microprocesseur.

20. Procédé selon l'une quelconque des revendications 3, 8, 18 ou 19, comprenant en outre le retrait du moule avec la formulation retenue du montage.

21. Procédé selon une quelconque revendication précédente, dans lequel la formulation comprend au moins un polymère dissous dans un solvant, le procédé comprenant en outre le retrait du solvant de la formulation retenue dans chacune de la pluralité de cavités.
